(19) Europäisches Patentamt
European Patent Office
Office européen des brevets

(11) Numéro de publication: **0 274 306**
**A2**

(12) **DEMANDE DE BREVET EUROPEEN**

(21) Numéro de dépôt: **87402755.0**

(51) Int. Cl.⁴: **C07D 251/34**

(22) Date de dépôt: **04.12.87**

(30) Priorité: **11.12.86 FR 8617330**

(43) Date de publication de la demande:
**13.07.88 Bulletin 88/28**

(84) Etats contractants désignés:
**AT BE CH DE ES GB GR IT LI LU NL SE**

(71) Demandeur: **Société Chimique des Charbonnages S.A.**
**Tour Aurore Place des Reflets**
**F-92080 Paris La Défense 2 Cédex 5(FR)**

(72) Inventeur: **Teissier Rémy**
**4, rue des Couteliers**
**F-31000 Toulouse(FR)**
Inventeur: **Clamens Serge**
**19, rue de la Camargue**
**F-31170 Tournefeuille(FR)**

(74) Mandataire: **Rieux, Michel**
**SOCIETE CHIMIQUE DES CHARBONNAGES S.A. Service Propriété Industrielle Tour Aurore Place des Reflets Cédex no. 5**
**F-92080 Paris la Défense 2(FR)**

(54) **Dichlorhydrine de l'acide cyanurique, ses dérivés trisubstitués et leur procédé de préparation.**

(57) L'invention a pour objet de nouveaux dérivés (I) de l'acide cyanurique de formule

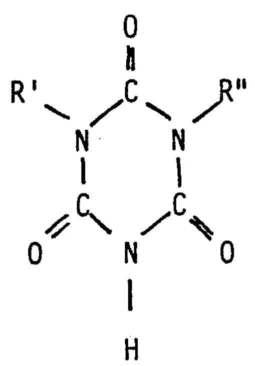

dans laquelle
- R′ et R″ sont choisis parmi $-CH_2-CHCl-CH_2OH$ et $-CH_2-CH(OH)-CH_2Cl$, et leur procédé de préparation.

Les dérivés (I) peuvent être utilisés comme agents réticulants dans les polymères. Ils peuvent également servir à la préparation de dérivés trisubstitués de l'acide cyanurique.

EP 0 274 306 A2

La présente invention concerne de nouveaux dérivés de l'acide cyanurique qui sont les 1-3 di-(chloro hydroxy propyle) isocyanurates, plus communément appelés dichlorhydrine de l'acide cyanurique, leur procédé ce préparation et leur application à la préparation de dérivés trisubstitués de l'acide cyanurique.

On connaît de nombreux dérivés organiques de l'acide cyanurique. Ce sont des dérivés triaziniques substitués sur l'azote de formule :

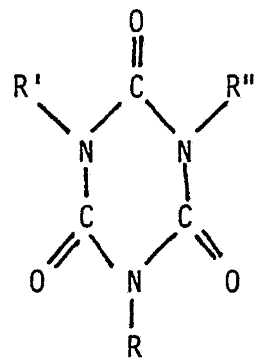

On connaît notamment la 1-3-5 tri(3 chloro 2 hydroxy propyle) isocyanurate dans lequel :

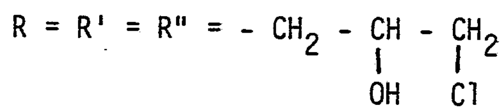

Il peut être utlisé comme intermédiaire réactionnel dans la préparation du triglycile isocyanurate. Il peut être obtenu par réaction de l'acide cyanurique sur l'épichlorhydrine (se reporter au brevet japonais N°75.160287). Il peut aussi être préparé par réaction de l'acide cyanurique, du formol et de l'épichlorhydrine (se reporter au brevet japonais N° 99060/1975).

Parmi les dérivés disubstitués connus de l'article isocyanurique, on peut citer le dihydroxyéthyle isocyanurate dont aucune fabrication satisfaisante n'a été mise au point, que ce soit à partir d'acide cyanurique et d'oxyde d'éthylène ou à partir d'acide cyanurique et de chlorhydrine de glycol. En fait, il est isolé comme sous-produit de la fabrication du tris-hydroxyéthyle isocyanurate.

La présente invention a pour premier objet de nouveaux dérivés de l'acide cyanurique de formule :

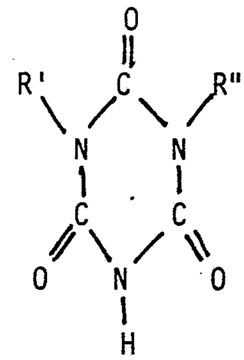

dans laquelle :

-R' et R" sont choisis parmi les groupes chlorhydrines

$-CH_2-CHCl-CH_2OH$ et $-CH_2-CH(OH)-CH_2Cl$.

Le spectre infra rouge (figure 1) et le spectre de masse (figure 2) confirment la structure des dérivés (I), à savoir le 1-3 di(chloro hydroxy propyle) 1-3-5 triazine (1H, 3H, 5H) 2-4-6 trione, plus communément appelé dichlorhydrine de l'acide cyanurique. Sa forumule brute est $C_9H_{13}Cl_2N_3O_5$. Son poids moléculaire est de 314,25. A température ambiante, il se présente comme un liquide visqueux blanchâtre. Il est soluble dans l'eau, le méthanol, l'éthanol et le diméthylformaldéhyde. Par contre, il est peu soluble dans l'acétone, le chloroforme et l'acétate d'éthyle.

La dichlorhydrine de l'acide cyanurique (I) selon l'invention peut être préparée de diverses manières. Selon un premier mode de fabrication, on peut utiliser la méthode dite de Detoeuf à la chlorurée (décrite dans le Bulletin Société Chimique 1922-31 pages 169-176). Selon un second mode de fabrication, on peut faire réagir la diallyle isocyanurate avec du chlore en présence d'oxyde mercurique (HgO), conformément à la méthode décrite dans Organic-Synthèse-Collective-Volume I à la page 158. Selon un troisième mode de fabrication, on fait réagir le diallyle isocyanurate qui est en suspension dans l'eau avec de l'acide triichloroisocyanurique. En effet, l'acide trichloroisocyanurique (ci-après dénommé ATCC) se décompose au contact de l'eau pour donner l'acide hypochloreux (HOC1) et l'acide isocyanurique (ci-après dénommé AC), l'acide hypochloreux ainsi libéré réagit rapidement avec le diallyle isocyanurate en suspension dans l'eau. La réaction est généralement menée à une température de l'ordre de 5-22°C et sous la pression atmosphérique.

De préférence, on utilise un rapport molaire d'ATCC sur le diallyle isocyanurate tel que le rapport molaire de chlore actif (C1 [1]) sur les liaisons éthyléniques soit égal à 1 ou supérieur de manière à bénéficier d'un léger excès de chlore actif (C1 [1])

De préférence encore, en raison de l'exothermicité de la réaction, on prévoit pendant la réaction un dispositif de refroidissement de manière à ce que la température du milieu réactionnel n'excède pas 22°C.

En fin de réaction, lorsque tout le chlore actif de l'ATCC a été consommé, on récupère l'acide cyanurique libéré par filtration. Après extraction et purification on recueille la dichlorhydrine de l'acide cyanurique qui est un produit visqueux clair qui se fige à température ambiante. Les résultats des analyses chimiques et des analyses physico-chimiques (acidimétrie, teneur en chlore libre : C1, teneur en chlore hydrolysable, analyse élémentaire de C, N, O, H, résonance magnétique nucléaire du proton (RMN[1]H), chromatographie sur cellulose spectre de masse (se reporter à la figure 2) et spectre infra rouge (se reporter à la figure 1) confirment la structure du produit (1).

Ce dernier mode de fabrication, par rapport aux deux premiers indiqués ci-dessus, présente l'avantage de ne pas utiliser de chlore gazeux. De plus, conformément à ce dernier mode, il est possible de regénérer l'ATCC à partir de l'acide isocyanurique recueilli en fin de réaction et de le recycler.

Les différents modes de fabrication précités permettent de préparer un mélange d'isomères de position qui sont des dichlorhydrines de l'acide cyanurique (I).

Ces isomères peuvent être séparés par chromatographie liquide préparative à haute performance.

La dichlorhydrine de l'acide cyanurique est avantageuse à deux titres :

-ce composé est utilisable comme réticulant dans les polymères tels que les polyoléfines, les polyacryliques, les polystyrènes, les polyallyliques ainsi que les polycondensats tels que les polyesters et les époxydes.

-de plus, ce composé est un intermédiaire réactionnel très intéressant dans la fabrication des dérivés (II) trisubstitués de l'acide cyanurique.de formule:

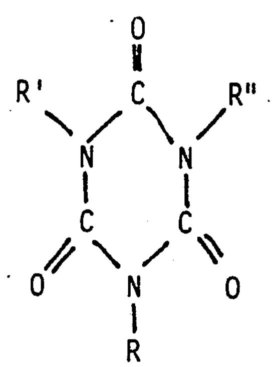

dans laquelle

-R′ et R″ sont choisis parmi les groupes réactifs glycidyles chlorhydrines

$-CH_2-CH(OH)-CH_2C1$ ou $-CH_2-CH\ C1-CH_2OH$

-et R est un groupe alkyle, un groupe aryle ou une fonction polymérisable telle qu'un groupe acrylique, méthacrylique ou allylique.

Les dérivés (II) se présentent généralement comme des produits visqueux incolores qui cristallisent à basse température, à environ - 30°C

Les dérivés (II) peuvent être utilisés comme réticulants dans les polymères tels que les polyoléfines, les polyacryliques, les polystyrènes, les polyallyliques, ainsi que les polycondensats tels que les polyesters et les époxydes. Il confèrent à ces polymères une bonne résistance à la chaleur et aux U.V. De plus, ils rendent ces polymères hydrosolubles. Les dérivés trisubstitués (II) qui comportent un radical R polymérisable peuvent également être polymérisés ou copolymérisés avec des monomères tels que les monomères (méth)-acryliques, les monomères allyliques, les monomères éthyléniques tels que l'éthylène, le propylène, le chlorure de vinyle, le styrène. De plus, les dérivés (I) et les dérivés (II) de l'acide cyanurique peuvent servir à la préparation de dérivés diglycides respectivement di-et tri-substitués de l'acide cyanurique par une réaction de déhydrochloration respectivement des dérivés (1) et des dérivés (II), ou à la préparation de dérivés dibishydroxy respectivement di-et tri-substitués de l'acide cyanurique par une réaction d'hydrolyse respectivement des dérivés (I) et des dérivés (II).

Pour la préparation des dérivés trisubstitués (II), on peut songer à utiliser différentes méthodes, notamment celles qui utilisent la dichlorhydrine de l'acide cyanurique comme produit de départ. Toutefois, parmi ces dernières, celles qui font intervenir l'action d'une base forte telle que NaOH ou KOH sont à bannir. En effet, les groupements chlorhydrines s'hydrolysent facilement en présence d'une base forte pour donner les groupements bishyroxy $-CH_2-CH(OH)-CH_2OH$.

Aussi, on prépare les dérivés trisubstitués (II) en faisant réagir la dichlorhydrine de l'acide cyanurique (I) avec, selon le cas, un halogénure d'alkyle ou un halogénure d'aryle en milieu aqueux et en présence d'au moins un catalyseur de type de transfert de phase, par exemple les ammoniums quaternaires, à une température comprise entre 50 et 140°C.

La réaction est généralement effectuée à une température comprise entre 50 et 100°C. De préférence, elle est menée à la température de reflux de l'halogénure ou à celle de l'eau dans le cas où la température d'ébullition de l'halogénure est supérieure à 100°C. La réaction peut également être effectuée à une température com-

prise entre 100 et 140°C, mais dans un autoclave. Au-delà de 140°C, les dérivés (II) de l'acide cyanurique se dégradent à une température inférieure à 50°C la cinétique de réaction est très faible.

En fin de réaction, on isole et purifie le dérivé trisubstitué (II) obtenu par extraction à l'aide d'un solvant tel que l'épichlorhydrine ou le formol. De préférence, on utilise un rapport molaire de dichlorhydrine de l'acide cyanurique sur l'halogénure, égal à 1 ou légèrement inférieur de manière à bénéficier d'un léger excès d'halogénure.

De préférence encore, dans le but d'avoir un milieu réactionnel de faible viscosité, la réaction est effectuée avec des concentrations aqueuses en dichlorhydrine de l'acide cyanurique et en halogénure n'excédant pas 0,5 mole/litre.

Parmi les halogénures utilisables dans le procédé selon l'invention, on peut citer le chlorure d'allyle, le bromure d'allyle, le chlorure de benzyle, l'acrylate de chloroéthyle, l'acrylate de chlorométhyle, le méthacrylate de chloroéthyle, le méthacrylate de chlorométhyle.

Parmi les catalyseurs utilisables dans le procédé selon l'invention, on peut citer, par exemple, le chlorure de tétraéthyle ammonium, le bromure de tétraéthyle ammonium, le chlorure de tétrathyle benzyle ammonium, le bromure de tétraéthyle benzyle ammonium, les amines tertiaires telles que la triméthyle amine, la triéthyle amine ou les sels de phosphonium quaternaire tels que les sels de phosphonium quaternaire, par exemple dans un rapport molaire par rapport à la dichlorhydrine de l'acide cyanurique compris entre $10^{-2}$ et $10^{-1}$.

**EXEMPLE 1** (Synthèse du 1,3 di-(chlorohydroxypropyle) isocyanurate)

A une suspension agitée de 1/2 mole de diallyle isocyanurate (104,5g) dans 2 litres d'eau maintenue entre 15 et 20°C, on ajoute par fractions successives 78g d'acide trichlorocyanurique soit 0,335 mole.

On maintient la température entre 15 et 20°C.

Après le dernier ajout d'acide trichlorocyanurique on laisse en contact 6 heures.

La suspension commence à se clarifier puis un nouveau précipité blanc apparaît.

Au bout de 6 heures on dose le chlore actif résiduel : 0,5g dans le mélange réactionnel que l'on déplace par un excès d'eau oxygénée.

On filtre et on récupère 41g d'acide cyanurique soit 95 % de la théorie.

On concentre le filtrat sous vide et on récupère à nouveau l'acide cyanurique (2g) ce qui correspond à un taux de réduction de 100 %. C'est ce mélange réactionnel (A) qui sera utilisé ensuite dans les exemples III et IV pour la synthèse respectivement du 1,3-di(chlorohydroxypropyle)5 monobenzyle isocyanurate).

On extrait 2 fois àl'épichlorhydrine.

On concentre la phase épichlorhydrine et on termine la concentration par des entraînements à la vapeur d'eau.

On termine de purifier le produit 24 heures à l'étuve sous vide.

On récupère 134g d'huile claire qui fige peu à peu à température ambiante.

Ce produit est très hygroscopique.

Le rendement par rapport au diallyle isocyanurate est de 85 %.

Les résultats des analyses chimiques et physico-chimiques sont les suivants :

-Acidimétrie : 93 % de la théorie

-teneur en C1 : 0,29 % en poids (obtenu par argentimétrie)

-teneur en chlorure hydrolysable : 22,5 % (obtenu par hydrolyse à reflux durant 2 H dans NaOH(5N) puis dosage argentimétrique des ions C1)

-Analyse élémentaire :

C : 34,48 % (valeur théorique : 34,39 %)

H : 4,28 % (valeur théorique : 4,14 %)

N : 13,71 % (valeur théorique : 13,38 %)

O : 25,76 % (valeur théorique : 25,40 %)

-Spectre infra-rouge : se reporter à la ligne 1

-Spectre de masse : se reporter à la figure 2

-chromatographie sur cellulose : un seul spot révélé à l'iode

(-éluant : méthanol pur

- éluant : méthanol/acétate de méthyle 80/20)

**EXEMPLE II** (synthèse du 1,3 di-(chlorohydroxypropyle) isocyanurate.)

Dans un réacteur de 5 litres, on ajoute 800 g de glace pilée à une solution de 25 g de chlorure mercurique dans 500cc d'eau ; puis on ajoute une solution refroidie de 190 g de NaOH dans 500cc d'eau, et on fait passer à travers le mélange un courant rapide de chlore. Ce mélange doit être maintenu à 5°C au plus. On arrête l'addition de chlore lorsque le précipité jaune d'oxyde mercurique a disparu. Alors 1600cc d'acide nitrique froid (1,5N) est rajouté doucement sous agitation.

On détermine la concentration d'acide hypochloreux par oxydation d'une solution de KI en milieu chlorhydrique et titration en retour par du thiosulfate.

On doit trouver une quantité de HOC1 comprise entre 3,5 et 4 %.

On recueille la quantité nécessaire pour traiter

une demi mole de diallyle isocyanurate (104,5g).

On ajoute par fractions successives la solution d'acide hypochloreux à la suspension de diallyle isocyanurate dans l'eau (2'). On maintient la température entre 15°C et 20°C.

Peu à peu le diallyle isocyanurate se solubilise.

On laisse en contact après le dernier ajout de HOC1 pendant 6 heures.

On dose le chlore actif (KI - Thiosulfate). Il reste 0,5 g environ de CL ' dans le milieu que l'on déplace par un excès de $H_2O_2$.

On concentre le milieu puis on extrait par de l'épichlororhydrine.

On récupère la phase épichlorhydrine que l'on concentre sous vide.

On termine la concentration par des entaînements à la vapeur d'eau.

On obtient une huile claire très hygroscopique : la dichlorhydrine de l'acide cyanurique avec un rendement de 53 %.

Les spectres de masse et infra-rouge sont identiques à ceux obtenus dans l'exemple 1 (se reporter respectivement aux figures 2 et 1).

**EXEMPLE III** (Synthèse du 1,3 di-(chlorohydroxypropyle) 5 monoallyle isocyanurate.

On reprend le mélange réactionnel (A) préparé ci-dessus.

Au mélange réactionnel (A) obtenu ci dessus, on ajoute :

-39 g (0,51 mole) de chlorure d'allyle,

-5 g de tétraéthylammonium de chlorure.

On porte le tout à reflux qui débute vers 47°C. Au bout de 12 heures de réaction, la température de reflux est à 95°C. Le chlorure d'allyle a disparu (pas de phase organique).

On laisse refroidir.

On dose par argentimétrie (dosage à l'aide de $AgNO_3$) les chlorures apparus. Cela correspond à un taux de réaction de 90 %. Par chromatographie gaz, on dose le chlorure d'allyle restant. Ce chlorure d'allyle restant donne un taux de réaction de 95 % environ.

On extrait trois fois par 300 cc de chloroforme. On réunit les 3 fractions organiques que l'on sèche par $CaCl_2$.

On concentre le milieu.

Puis on le porte à -30°C. Un produit cristallise que l'on filtre rapidement et que l'on sèche sous vide à température ambiante.

On récupère 83 g d'un produit pâteux assez hygroscopique, blanc jaune qui est le 1-3 di-(chlorohydroxypropyle) 5 monoallyle isocyanurate.

Le produits est identifié comme suit :

Le spectre RMN $C^{13}$ présente 3 pics nouveaux par rapport à celui de la dichlorhydrine de l'acide cyanurique :

2 pics entre 130 et 140 ppm correspondant au motif - C = CH₂

1 pic vers 50 ppm correspondant à -N-$CH_2$

Le dosage de chlore hydrolysable (2h à reflux dans NaOH 5N et dosage des $C1^{(-)}$ par argentimétrie) donne un taux de chlore de 20,5 % (théorie 20,06 %).

Le spectre infra-rouge du 1,3 di-(chlorohydroxypropyle)5 monoallyle isocyanurate est reporté en annexe sur la figure 3.

Le spectre infra-rouge présente des bandes supplémentaires entre 100 et 1200 $cm^{-1}$ par rapport au spectre de la dichlorhydrine qui sont les bandes de vibration de l'allyle (se reporter à la figure 3).

Une analyse élémentaire donne les résultats suivants :

C : 39,78 % (Théorie : 40,68 %)

H : 4,92 % (Théorie : 4,80 %)

N : 11,44 % (Théorie : 11,86 %)

O : 22,96 % (Théorie : 22,60 %)

Ce produit, 1-3 di(chlorhydroxypropyle) 5 monoallyle isocyanurate est un nouveau dérivé de l'acide cyanurique, monomère allylique, hydrosoluble, post réticulable.

En effet, le chlore en position 1-3 est très labile :

-par déshydrochloration, il donne :

. le 1-3 Di glycidyle monoallyle isocyanurate

-par hydrolyse, il donne :

. le 1-3 Di (bi hydroxypropyl) monoallyle, poly allyle, ...

Ce monomère, et ses dérivés trouve une application dans les matériaux polymère du type : poly oléfines, poly acrylique, poly allyle, ...

**EXEMPLE IV** (Synthèse du 1,3 di (chlorohydroxypropyle) 5 monobenzyle isocyanurate)

Au mélange réactionnel (A) décrit dans l'exemple (I), on ajoute :

-64 g de chlorure de benzyle (0,506 mole)

-5 g de tétraéthylammonium chlorure.

On porte durant 24 heures le mélange réactionnel à 100°C.

Au bout de 24 heures, on laisse refroidir.

Le dosage par argentimètre des chlorures indique un taux de réaction de 65 %.

On extrait à 3 reprises par 300cc de chloroforme.

On concentre la phase organique préalablement séchée.

Un produit cristallise. On récupère 72 g de 1-3 di (chlorohydroxypropyle isocyanurate) 5 monobenzyle isocyanurate soit un rendement de 35,6 % par rapport au diallyle isocyanurate.

Ce produit est identique par analyse élémentaire.

C : 46,63 % (Théorie : 47,52 %)
H : 4,82 % (Théorie : 4,70 %)
N : 9,75 % (Théorie : 10,40 %)
O : 20,35 % (Théorie : 19,80 %)
Un dosage de chlore hydrolysable donne un résultat de 17,9 % (Théorie : 17,58 %)

Le spectre infra-rouge est proche de celui de la dichlorhydrine de l'acide cyanuraique, avec des bandes supplémentaire dans la carte d'identité de la molécule (1000 $cm^{1}$ à 1500 $c^{m-1}$).

Ce produit trouve une application dans la post-réticulation des matériaux polymères, du fait de son chlore labile.

**EXEMPLE V** (comparatif)

Nous avons essayé de faire un sel sodique de la dichlorhydrine de l'acide cyanurique, destiné à être ensuite mis en présence d'un chlorure d'alkyle.

Dans un litre de méthanol, on dissout 10g de NaOH puis on introduit 78g (0,248 mole) de dichlorydrine de l'acide cyanurique.

On laisse en contact durant 3 heures; puis on distille le méthanol.

On récupère un produit pâteux demi-solide qui présente un taux de chlorure de 5,5 % en poids.

Il y a donc un déplacement partiel du chlore de la dichlorhydrine.

Un essai dans l'eau donne un taux de chlore de 11,3 %.

Un essai de l'éthanol absolu donne un produit avec un taux de chlorure de 3,6 %.

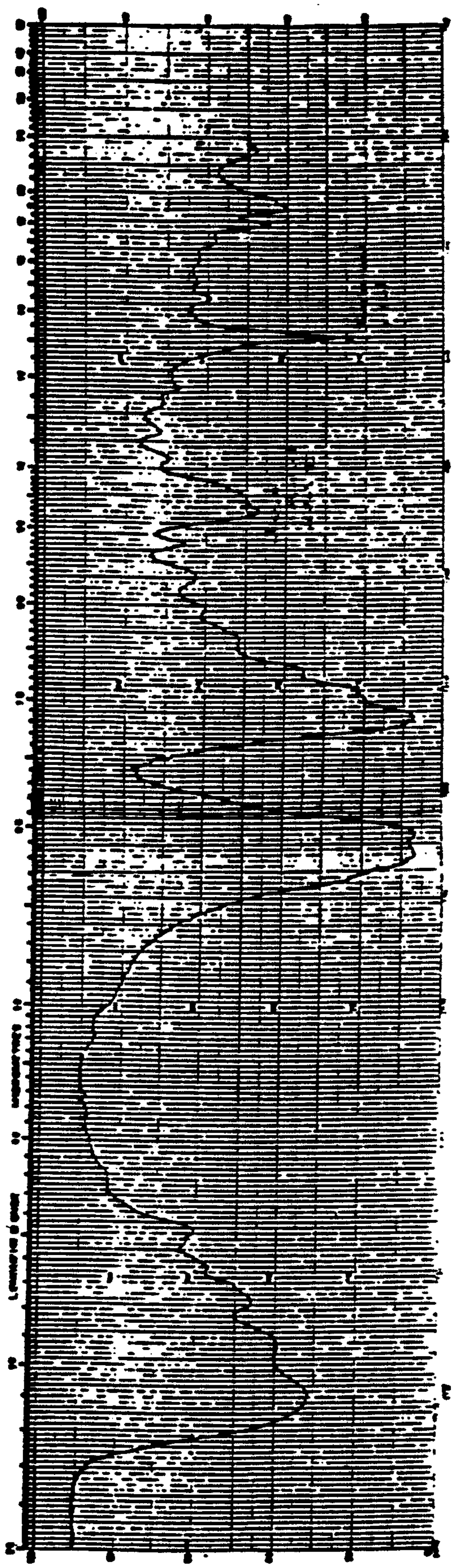

FIGURE 1

SPECTRE DE MASSE

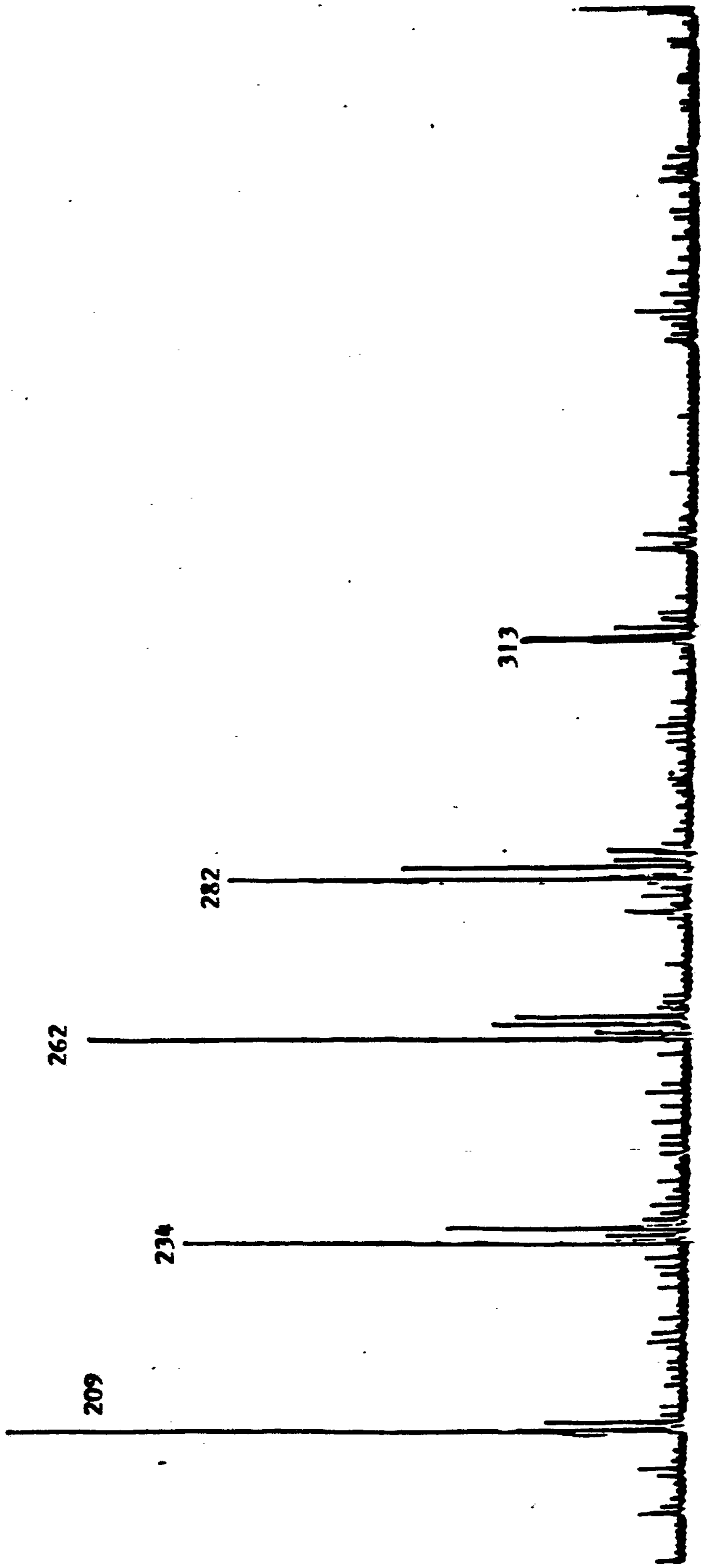


FIGURE 2

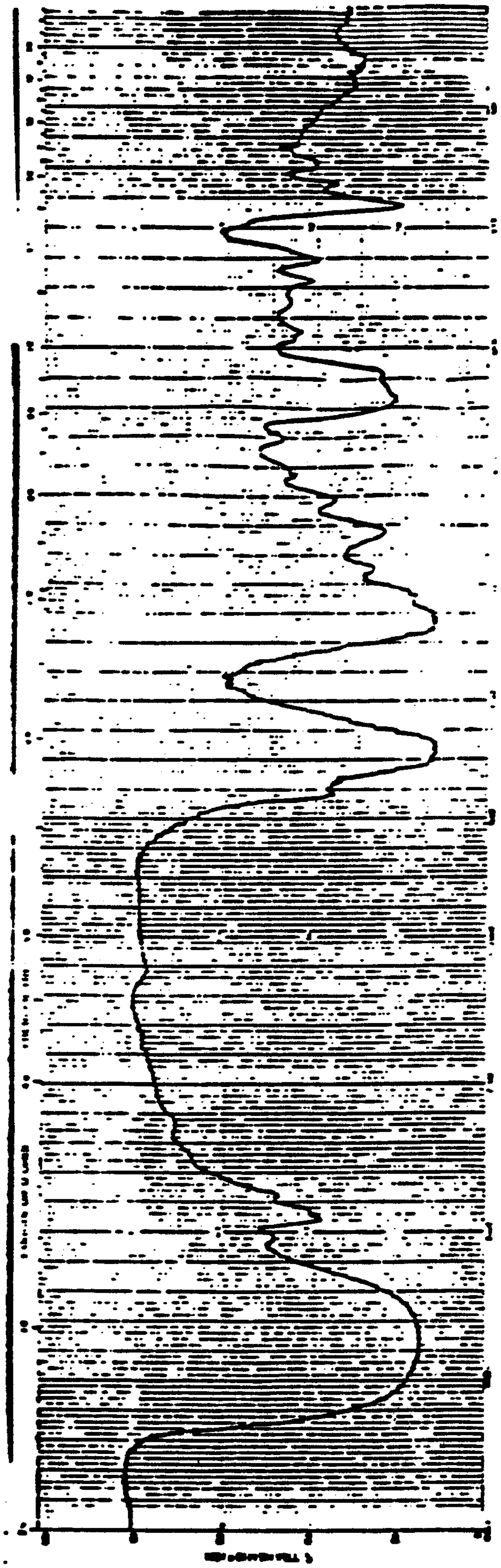

FIGURE 3

## Revendications

1. Dérivés de l'acide cyanurique de formule (1)

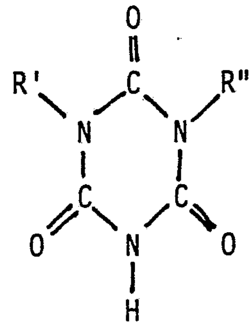

dans laquelle :

-R′ et R″ sont choisis parmi les groupes chlorhydrines

$-CH_2-CHCl-CH_2OH$ et $-CH_2-CH(OH)-CH_2Cl$

2. Procédé de préparation des dérivés définis dans la revendication 1 caractérisé en ce que l'on fait réagir le diallyle isocyanurate qui est en suspension dans l'eau avec de l'acide trichloro-isocyanurique, et en ce que l'on récupère ensuite le dérivé de formule (I) ainsi formé.

3. procédé selon la revendication 2, caractérisé en ce que la température de réaction n'excède pas 22°C.

4. Application des dérivés (I) définis dans la revendication 1 à la préparation des dérivés trisubstitués de l'acide cyanurique de formule (II) :

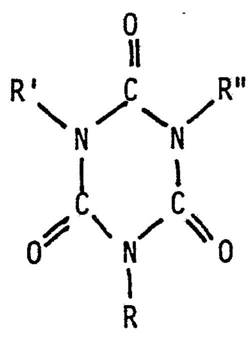

dans laquelle :

-R′ et R″ sont choisis parmi les groupes chlorhydrines

$-CH_2-CHCl-CH_2OH$ et $-CH_2-CH(OH)-CH_2Cl$,

-et R est un groupe alkyle, un groupe aryle ou une fonction polymérisable telle qu'un groupe acrylique, méthacrylique ou allylique.

On concentre le milieu puis on extrait par de l'épichlororhydrine.

5. Procédé de préparation des dérivés de formule (II) définis dans la revendication 4, caractérisé en ce que l'on fait réagir le dérivé (1) défini dans la revendication 1 avec, selon le cas, un halogénure d'alkyle ou un halogenure d'aryle, en présence d'au moins un catalyseur de transfert de phase et à une température comprise entre 5O et 14O°C, puis on sépare le dérivé (II) ainsi obtenu.

6. Application des dérivés (I) et des dérivés (II) à la préparation de dérivés diglycidyles respectivement di et trisubstitués de l'acide cyanurique par une réaction de déhydrochoration respectivement des dérivés (I) et des dérivés (II).

7. Aplication des dérivés (I) et des dérivés (II) à la préparation de dérivés dibishydroxys respectivement di et trisubstitués de l'acide cyanurique par une réaction d'hydrolyse respectivement des dérivés (I) et (II).